# EUROPEAN PATENT APPLICATION

(11) **EP 3 936 138 A1**
(43) Date of publication of application: **12.01.2022**
(21) Application number: 20382617.7
(22) Date of filing: 09.07.2020
(51) Int. Cl.: A61K 35/618, A61K 38/08, A61K 38/10, A61P 17/00

(54) **PHARMACEUTICAL COMPOSITION FOR THE TREATMENT OF VITILIGO**

(71) Applicant: Bella Aurora Labs, S.A., 08690 Santa Coloma de Cervelló (Barcelona) (ES); Bordignon, Matteo, 35126 Padova (IT)
(72) Inventor: BORDIGNON, Matteo, 35126 Padova (IT); HERNÁNDEZ NAVARRO, Sergi, 08690 Santa Coloma de Cervelló (BARCELONA) (ES); SEGURA TEJEDOR, Jordi, 08690 Santa Coloma de Cervelló (BARCELONA) (ES)
(74) Representative: Pons

(57) **Abstract**

The present invention relates to a pharmaceutical composition for the treatment of vitiligo comprising the combination of a peptide which is an inhibitor of the melanoma inhibitory activity (MIA) protein and snail secretion. The invention also relates to a kit comprising this composition and a dietary supplement comprising an antioxidant. The invention also relates to the use of the composition or to the use of the kit comprising such composition for the prevention and/or treatment of vitiligo.

SEQUENCE LISTING

<110> Bella Aurora Labs, SA
      Matteo Bordignon

<120> Pharmaceutical composition for the treatment of vitiligo

<130> EP4007.2

<160> 50

<170> BiSSAP 1.3.6

<210> 1
<211> 7
<212> PRT
<213> Artificial Sequence


<220>
<223> MIA inhibitor

<400> 1
Val Pro His Ile Pro Pro Asn
1               5

<210> 2
<211> 7
<212> PRT
<213> Artificial Sequence


<220>
<223> MIA inhibitor

<400> 2
Met Pro Pro Thr Gln Val Ser
1               5

<210> 3
<211> 7
<212> PRT
<213> Artificial Sequence


<220>
<223> MIA inhibitor

<400> 3
Gln Met His Pro Trp Pro Pro
1               5

<210> 4
<211> 7
<212> PRT
<213> Artificial Sequence


<220>
<223> MIA inhibitor

<400> 4
Gln Pro Pro Phe Trp Gln Phe

1                   5

<210> 5
<211> 7
<212> PRT
<213> Artificial Sequence

<220>
<223> MIA inhibitor

<400> 5
Thr Pro Pro Gln Gly Leu Ala
1               5

<210> 6
<211> 7
<212> PRT
<213> Artificial Sequence

<220>
<223> MIA inhibitor

<400> 6
Ile Pro Pro Tyr Asn Thr Leu
1               5

<210> 7
<211> 7
<212> PRT
<213> Artificial Sequence

<220>
<223> MIA inhibitor

<400> 7
Ala Val Arg Pro Ala Pro Leu
1               5

<210> 8
<211> 7
<212> PRT
<213> Artificial Sequence

<220>
<223> MIA inhibitor

<400> 8
Gly Ala Lys Pro His Pro Gln
1               5

<210> 9
<211> 7
<212> PRT
<213> Artificial Sequence

<220>
<223> MIA inhibitor

<400> 9
Gln Gln Leu Ser Pro Leu Pro
1               5

<210> 10
<211> 7
<212> PRT
<213> Artificial Sequence

<220>
<223> MIA inhibitor

<400> 10
Gly Pro Pro Pro Ser Pro Val
1               5

<210> 11
<211> 7
<212> PRT
<213> Artificial Sequence

<220>
<223> MIA inhibitor

<400> 11
Leu Pro Leu Thr Pro Leu Pro
1               5

<210> 12
<211> 12
<212> PRT
<213> Artificial Sequence

<220>
<223> MIA inhibitor

<400> 12
Gln Leu Asn Val Asn His Gln Ala Arg Ala Asp Gln
1               5                   10

<210> 13
<211> 12
<212> PRT
<213> Artificial Sequence

<220>
<223> MIA inhibitor

<400> 13
Thr Ser Ala Ser Thr Arg Pro Glu Leu His Tyr Pro
1               5                   10

<210> 14
<211> 12
<212> PRT
<213> Artificial Sequence

<220>

<223> MIA inhibitor

<400> 14
Thr Phe Leu Pro His Gln Met His Pro Trp Pro Pro
1               5                   10

<210> 15
<211> 12
<212> PRT
<213> Artificial Sequence

<220>
<223> MIA inhibitor

<400> 15
Val Pro His Ile Pro Pro Asn Ser Met Ala Leu Thr
1               5                   10

<210> 16
<211> 12
<212> PRT
<213> Artificial Sequence

<220>
<223> MIA inhibitor

<400> 16
Arg Leu Thr Leu Leu Val Leu Ile Met Pro Ala Pro
1               5                   10

<210> 17
<211> 12
<212> PRT
<213> Artificial Sequence

<220>
<223> MIA inhibitor

<400> 17
Tyr Asn Leu Pro Lys Val Ser Ser Asn Leu Ser Pro
1               5                   10

<210> 18
<211> 12
<212> PRT
<213> Artificial Sequence

<220>
<223> MIA inhibitor

<400> 18
Met Pro Pro Thr Gln Val Ser Lys Phe Arg Leu Ile
1               5                   10

<210> 19
<211> 12
<212> PRT
<213> Artificial Sequence

<220>
<223> MIA inhibitor

<400> 19
Ala Asn Ile Asp Ala Thr Pro Leu Phe Leu Arg Ala
1               5                   10

<210> 20
<211> 12
<212> PRT
<213> Artificial Sequence


<220>
<223> MIA inhibitor

<400> 20
Leu Leu Arg Thr Thr Glu Thr Leu Pro Met Phe Leu
1               5                   10

<210> 21
<211> 7
<212> PRT
<213> Artificial Sequence


<220>
<223> MIA inhibitor

<400> 21
Ser Ala Leu Glu Pro Leu Val
1               5

<210> 22
<211> 7
<212> PRT
<213> Artificial Sequence


<220>
<223> MIA inhibitor

<400> 22
Gly Ser Pro Thr Pro Asn Ala
1               5

<210> 23
<211> 7
<212> PRT
<213> Artificial Sequence


<220>
<223> MIA inhibitor

<400> 23
Ala Pro Ser His Ala Thr His
1               5

<210> 24
<211> 7
<212> PRT

<213> Artificial Sequence

<220>
<223> MIA inhibitor

<400> 24
Thr Thr Val Gly His Ser Asp
1               5

<210> 25
<211> 7
<212> PRT
<213> Artificial Sequence

<220>
<223> MIA inhibitor

<400> 25
Thr His Phe Ser Thr Phe Thr
1               5

<210> 26
<211> 7
<212> PRT
<213> Artificial Sequence

<220>
<223> MIA inhibitor

<400> 26
Ser Leu Leu Leu Asp Thr Ser
1               5

<210> 27
<211> 12
<212> PRT
<213> Artificial Sequence

<220>
<223> MIA inhibitor

<400> 27
Ser Val Ala Met Lys Ala His Lys Pro Leu Leu Pro
1               5                   10

<210> 28
<211> 12
<212> PRT
<213> Artificial Sequence

<220>
<223> MIA inhibitor

<400> 28
Asn Thr Ile Pro Gly Phe Ala Ser Lys Ser Leu Asp
1               5                   10

<210> 29

<211> 12
<212> PRT
<213> Artificial Sequence

<220>
<223> MIA inhibitor

<400> 29
Val Ser Asn Tyr Lys Phe Tyr Ser Thr Thr Ser Ser
1               5                   10

<210> 30
<211> 12
<212> PRT
<213> Artificial Sequence

<220>
<223> MIA inhibitor

<400> 30
Val Ser Arg His Gln Ser Trp His Pro His Asp Leu
1               5                   10

<210> 31
<211> 12
<212> PRT
<213> Artificial Sequence

<220>
<223> MIA inhibitor

<400> 31
His Leu Asn Ile Leu Ser Thr Leu Trp Lys Tyr Arg
1               5                   10

<210> 32
<211> 12
<212> PRT
<213> Artificial Sequence

<220>
<223> MIA inhibitor

<400> 32
His Asn Ala Ser Pro Ser Trp Gly Ser Pro Val Met
1               5                   10

<210> 33
<211> 12
<212> PRT
<213> Artificial Sequence

<220>
<223> MIA inhibitor

<400> 33
Ser His Pro Trp Asn Ala Gln Arg Glu Leu Ser Val
1               5                   10

<210> 34
<211> 12
<212> PRT
<213> Artificial Sequence

<220>
<223> MIA inhibitor

<400> 34
His His Trp Pro Phe Trp Arg Thr Leu Pro Leu Ser
1               5                   10

<210> 35
<211> 12
<212> PRT
<213> Artificial Sequence

<220>
<223> MIA inhibitor

<400> 35
Trp His Thr Lys Phe Leu Pro Arg Tyr Leu Pro Ser
1               5                   10

<210> 36
<211> 12
<212> PRT
<213> Artificial Sequence

<220>
<223> MIA inhibitor

<400> 36
Asn Asn Thr Ser Phe Thr Val Val Pro Ser Val Pro
1               5                   10

<210> 37
<211> 12
<212> PRT
<213> Artificial Sequence

<220>
<223> MIA inhibitor

<400> 37
Ser His Leu Ser Thr Trp Lys Trp Trp Gln Asn Arg
1               5                   10

<210> 38
<211> 12
<212> PRT
<213> Artificial Sequence

<220>
<223> MIA inhibitor

<400> 38

Phe His Trp His Pro Arg Leu Trp Pro Leu Pro Ser
1               5                   10

<210> 39
<211> 12
<212> PRT
<213> Artificial Sequence

<220>
<223> MIA inhibitor

<400> 39
Trp His Trp Thr Tyr Gly Trp Arg Pro Pro Ala Met
1               5                   10

<210> 40
<211> 12
<212> PRT
<213> Artificial Sequence

<220>
<223> MIA inhibitor

<400> 40
Phe His Trp Arg Tyr Pro Leu Pro Leu Pro Gly Gln
1               5                   10

<210> 41
<211> 12
<212> PRT
<213> Artificial Sequence

<220>
<223> MIA inhibitor

<400> 41
Trp His Trp Pro Leu Phe Ile Pro Asn Thr Thr Ala
1               5                   10

<210> 42
<211> 12
<212> PRT
<213> Artificial Sequence

<220>
<223> MIA inhibitor

<400> 42
Trp His Asn Gly Ile Trp Trp His Tyr Gly Val Arg
1               5                   10

<210> 43
<211> 12
<212> PRT
<213> Artificial Sequence

<220>
<223> MIA inhibitor

<400> 43
His His Leu Asn Tyr Leu Trp Pro Trp Thr Arg Val
1               5                   10

<210> 44
<211> 12
<212> PRT
<213> Artificial Sequence

<220>
<223> MIA inhibitor

<400> 44
Phe Trp His Arg Trp Ser Thr Phe Pro Glu Gln Pro
1               5                   10

<210> 45
<211> 12
<212> PRT
<213> Artificial Sequence

<220>
<223> MIA inhibitor

<400> 45
Trp His Met Ser Tyr Phe Trp Thr Arg Pro Pro Gln
1               5                   10

<210> 46
<211> 12
<212> PRT
<213> Artificial Sequence

<220>
<223> MIA inhibitor

<400> 46
Phe His Leu Asn Trp Pro Ser Arg Ala Asp Tyr Leu
1               5                   10

<210> 47
<211> 12
<212> PRT
<213> Artificial Sequence

<220>
<223> MIA inhibitor

<400> 47
Trp His Lys Asn Thr Asn Trp Pro Trp Arg Thr Leu
1               5                   10

<210> 48
<211> 12
<212> PRT
<213> Artificial Sequence

<220>
<223> MIA inhibitor

<400> 48
Ala Leu Ser Pro Ser Gln Ser His Pro Val Arg Ser
1               5                   10

<210> 49
<211> 12
<212> PRT
<213> Artificial Sequence


<220>
<223> MIA inhibitor

<400> 49
Gly Thr Gln Ser Thr Ala Ile Pro Ala Pro Thr Asp
1               5                   10

<210> 50
<211> 13
<212> PRT
<213> Artificial Sequence


<220>
<223> MC1 receptor agonist; 4: Nleu; 7: DPhe; 12: DPro; 1: Acetylation;
      13: Amidation

<400> 50
Ser Tyr Ser Leu Glu His Phe Arg Trp Gly Lys Pro Val
1               5                   10

## Description

### Technical field

The present invention relates to a pharmaceutical composition for the treatment and/or prevention of vitiligo.

### State of the art

Vitiligo is an acquired skin disorder characterized by hypopigmented or depigmented areas and has a prevalence of about 0.5 to 2% in the world population.

Non-segmental vitiligo (NSV) is the most common clinical form of the disease, wherein the patches of depigmentation are usually bilateral and with some form of symmetry.

Vitiligo most commonly involves the face, digits, dorsal hands, flexor wrists, elbows, knees, shins, dorsal ankles, armpits, inguinal area, anogenital area, umbilicus, and nipples.

A loss of epidermal melanocytes is the pathologic hallmark of vitiligo, but the aetiology of the disease is multifactorial and still poorly understood. For example, among the factors which can contribute to non-segmental vitiligo are immunologic factors, oxidative stress, or a sympathetic neurogenic disturbance. On the other hand, it is thought that there is also some genetic predisposition to this disease (Taïeb et al., Vitiligo, N. Engl. J. Med., 2009, 360, 160-169).

Among the pharmacological treatments proposed so far are, for example, topical corticosteroids, topical macrolide immunomodulators, namely, the calcineurin inhibitors tacrolimus and pimecrolimus, and vitamin D3 analogues and antioxidants. On the other hand, the most used non-pharmacological treatment are ultraviolet radiations, both in the range of UVA and UVB (Guerra et al., Vitiligo: pathogenetic hypotheses and targets for current therapies, Current Drug Metabolism, 2010, 11, 451-467).

These available treatments are barely effective and some of them may involve some risk of adverse effects.

More recently, it has also been suggested the involvement of the protein MIA (*"Melanoma inhibitory activity"*) in the pathogenesis of non-segmental vitiligo based on the finding that, unlike in normal melanocytes, the MIA protein is expressed in vitiliginous skin and may cause the detachment of melanocytes (melanocytorrhagy) due to its interaction with alpha5beta1 integrin, thus perturbing the normal attachment of melanocytes to basal membrane (Bordignon et al., Role of alpha5beta1 integrin and MIA (melanoma inhibitory activity) in the pathogenesis of vitiligo, J. Dermatol. Sci., 2013, 71(2), 142-5). In an *in-vivo* mice model, it was confirmed that the injection of MIA in the tail of the animals induced vitiligo-like depigmentation in the injected area (Bordignon et al., MIA (melanoma inhibitory activity) is able to induce vitiligo in an in-vivo mice model by direct injection in the tail, Vitiligo International Symposium 2016, Meeting Abstracts).

Therefore, MIA is suggested as target for vitiligo therapy, and in the international patent application WO-A-2012/127352 a number of peptides and peptide derivatives which are MIA inhibitors are proposed for the prevention and treatment of vitiligo, by a mechanism involving the binding of the inhibitors to MIA and thus avoiding the interaction of MIA with alpha5beta1 integrins, which is the cause of melanocytorrhagy.

It is noticeable, however, that the document WO-A-2012/127352 does not provide any working example showing the effectiveness of those substances for vitiligo therapy and, actually, it does not give any hint about suitable methods or compositions for using those MIA inhibitors for effectively treating vitiligo.

Therefore, there is still the need to provide effective and safe compositions for the treatment and prevention of vitiligo.

### Object of the invention

The object of the present invention is a pharmaceutical composition comprising a MIA inhibitor and snail secretion.

Another aspect of the present invention is a kit comprising the pharmaceutical composition of the invention and a dietary supplement comprising an anti-oxidant.

Another aspect of the invention is such composition or said kit for use in the prevention and/or treatment of vitiligo.

### Description of the Drawings

Figure 1 shows three illustrative examples for testing the efficacy of three different compositions in a single patient, for comparative purposes.
Figure 2 shows a picture of one of the patients enrolled in the trial described in Example 4, before and after the treatment, wherein the left side of the face was treated with the composition A and the right side of the face was treated with the composition B.
Figure 3 shows a picture of one of the patients enrolled in the trial described in Example 4, before and after the treatment, who was treated with the composition C.

### Detailed description of the invention

The object of the present invention is a pharmaceutical composition comprising:
(a) a MIA inhibitor peptide which has a sequence selected from SEQ ID NO: 1 to SEQ ID NO: 49, or a derivative thereof;
(b) snail secretion; and
(c) at least one pharmaceutically acceptable excipient.

The authors of the present invention have developed a pharmaceutical composition comprising the combination of a MIA inhibitor peptide and snail secretion that, surprisingly, unlike the treatments available so far in the state of the art, is outstandingly effective for treating vitiligo.

Along the present description, as well as in the claims, the singular expressions, generally preceded by the articles "a", "an" or "the", are meant to include also the plural forms, unless the context clearly indicates otherwise. All percentages are expressed by weight, unless specifically stated otherwise. Numeric values preceded by the term "about" are meant to include also a certain variation around such value, namely a variation or ±5% of the stated amount. Numeric ranges defined by lower and upper endpoints are meant to include also said stated endpoints and they also include any narrower sub-range.

The amino acids are represented herein using the one-letter code, as is well-known by the skilled in the art, according to IUPAC-IUB Commission on Biochemical Nomenclature, as described in the book Biochemical Nomenclature and Related Documents, 2nd edition, Portland Press, 1992, London [ISBN 1-85578-005-4].

### MIA inhibitor peptide

One of the components of the composition of the invention is a substance having activity as inhibitor of the human melanoma inhibitory activity (MIA) protein, in particular, those disclosed in the international patent application WO-A-2012/127352, namely, a peptide having a sequence selected from SEQ ID NO: 1 to SEQ ID NO: 49:
- SEQ ID NO: 01 VPHIPPN
- SEQ ID NO: 02 MPPTQVS
- SEQ ID NO: 03 QMHPWPP
- SEQ ID NO: 04 QPPFWQF
- SEQ ID NO: 05 TPPQGLA
- SEQ ID NO: 06 IPPYNTL
- SEQ ID NO: 07 AVRPAPL
- SEQ ID NO: 08 GAKPHPQ
- SEQ ID NO: 09 QQLSPLP
- SEQ ID NO: 10 GPPPSPV
- SEQ ID NO: 11 LPLTPLP
- SEQ ID NO: 12 QLNVNHQARADQ
- SEQ ID NO: 13 TSASTRPELHYP
- SEQ ID NO: 14 TFLPHQMHPWPP
- SEQ ID NO: 15 VPHIPPNSMALT
- SEQ ID NO: 16 RLTLLVLIMPAP
- SEQ ID NO: 17 YNLPKVSSNLSP
- SEQ ID NO: 18 MPPTQVSKFRLI
- SEQ ID NO: 19 ANIDATPLFLRA
- SEQ ID NO: 20 LLRTTETLPMFL
- SEQ ID NO: 21 SALEPLV
- SEQ ID NO: 22 GSPTPNA
- SEQ ID NO: 23 APSHATH
- SEQ ID NO: 24 TTVGHSD
- SEQ ID NO: 25 THFSTFT
- SEQ ID NO: 26 SLLLDTS
- SEQ ID NO: 27 SVAMKAHKPLLP
- SEQ ID NO: 28 NTIPGFASKSLD
- SEQ ID NO: 29 VSNYKFYSTTSS
- SEQ ID NO: 30 VSRHQSWHPHDL
- SEQ ID NO: 31 HLNILSTLWKYR
- SEQ ID NO: 32 HNASPSWGSPVM
- SEQ ID NO: 33 SHPWNAQRELSV
- SEQ ID NO: 34 HHWPFWRTLPLS
- SEQ ID NO: 35 WHTKFLPRYLPS
- SEQ ID NO: 36 NNTSFTVVPSVP
- SEQ ID NO: 37 SHLSTWKWWQNR
- SEQ ID NO: 38 FHWHPRLWPLPS
- SEQ ID NO: 39 WHWTYGWRPPAM
- SEQ ID NO: 40 FHWRYPLPLPGQ
- SEQ ID NO: 41 WHWPLFIPNTTA
- SEQ ID NO: 42 WHNGIWWHYGVR
- SEQ ID NO: 43 HHLNYLWPWTRV
- SEQ ID NO: 44 FWHRWSTFPEQP
- SEQ ID NO: 45 WHMSYFWTRPPQ
- SEQ ID NO: 46 FHLNWPSRADYL
- SEQ ID NO: 47 WHKNTNWPWRTL
- SEQ ID NO: 48 ALSPSQSHPVRS
- SEQ ID NO: 49 GTQSTAIPAPTD
or a derivative thereof.

In one embodiment, the MIA inhibitor peptide has a sequence selected from SEQ ID NOs 12-20 and 27-49, preferably selected from SEQ ID NOs 34, 37, 39-43 and 45-47, and more preferably has the sequence SEQ ID NO: 40; or is a derivative thereof.

A derivative of a peptide having a sequence selected from SEQ ID NO: 1 to SEQ ID NO: 49 means a peptide with some small variations around these sequences, still retaining the MIA inhibitory activity. Examples of suitable variations are described below.

One suitable derivative is a peptide having one of the above sequences wherein one additional amino acid is present or wherein one amino acid of the sequence is deleted.

Another suitable derivative is a peptide wherein one or more of the amino acids of the sequence is/are substituted by another natural or non-natural amino acid, preferably no more than 3 amino acids of the sequence are substituted, preferably no more than 2 amino acids, and more preferably only one amino acid is substituted by another natural or non-natural amino acid.

Natural amino acids, as is well-known in the art, are those 20 amino acids which usually occur in natural proteins and peptides.

Also as is well-known in the art, non-natural (or unnatural or unusual) amino acids are based on natural amino acids but wherein one or more atoms are substituted with functional groups comprising up to 50 atoms selected from C, H, N, S, O, P, F, Cl, Br, I and Se. Suitable, non-limiting, examples of such non-naturally occurring amino acids are trans-3-methylproline, 2,4-methanoproline, cis-4-hydroxyproline, trans-4-hydroxyproline, *N*-methylglycine, allo-threonine, *N*-methylthreonine, hydroxyethylcysteine, hydroxyethylhomocysteine, nitroglutamine, homoglutamine, pipecolic acid, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into peptides or proteins.

Other suitable derivatives are those peptides of sequences 1 to 49, also including those having amino acid additions/deletions or substitutions, as discussed above, wherein at least one amino acid is modified, i.e., an additional moiety or functional group is added thereto. Such modifications may have the purpose of improving the stability, solubility and/or skin penetration of the peptide. Suitable modifications can be selected from glycosylation, acetylation (e.g. N-terminal), amidation (e.g. C-terminal), hydroxylation (e.g. hydroxyproline), carboxylation (e.g. gamma-carboxyglutamate), phosphorylation, alkylation, N-terminal lipidation (i.e., adding saturated or unsaturated C₁₂-C₁₈ fatty acids, such as myristic or palmitic, at the N-terminus, through an amide bond), or prenylation. Common modifications of the peptide sequences are acetylation of the N-terminus and amidation of the C-terminus. C-terminal amidation includes the formation of terminal amido groups of formula-CONH₂, -CONHR or -CONR₂, wherein R is typically a short (e.g. C₁-C₄) alkyl group. The simplest and most common C-terminal amido group is -CO-NH₂.

In a one embodiment, the MIA inhibitor peptide is a peptide which is acetylated at the N-terminus and/or amidated at the C-terminus, preferably is acetylated at the N-terminus and amidated at the C-terminus.

In a particularly preferred embodiment, the MIA inhibitor is a peptide:
- selected from SEQ ID NO: 1 to SEQ ID NO: 49, preferably selected SEQ ID NOs 12-20 and 27-49, more preferably selected from SEQ ID NOs 34, 37, 39-43 and 45-47, and still more preferably is a peptide of SEQ ID NO: 40; and
- wherein at least one amino acid of the sequence is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation, preferably the sequence is acetylated at the N-terminus and/or amidated at the C-terminus, and more preferably is acetylated at the N-terminus and amidated at the C-terminus.

The "MIA inhibitor peptide" or "MIA inhibitor" terms, as used interchangeably herein, refer widely to the MIA inhibitor substance used in the composition, either if it is strictly a peptide sequence or a modified sequence as disclosed above.

Those peptides and derivatives thereof can be prepared following standard methods in peptide synthesis, typically, by solid phase synthesis, or may be obtained commercially from several sources.

The composition of the invention may comprise one or more MIA inhibitor peptide, i.e. a single MIA inhibitor substance or the combination of two or more MIA inhibitors. When more than one MIA inhibitor is used, they may be used in any proportion and, therefore, the amount of MIA inhibitor as stated herein means the total amount of MIA inhibitor substances contained in the composition.

As disclosed in the international patent application WO-A-2012/127352 or in the international patent application WO-A-03/064457 or in the article Stoll et al., The extracellular human melanoma inhibitory activity (MIA) protein adopts an SH3 domain-like fold, EMBO J., 2001, 20(3), 340-349, the activity as MIA inhibitors of the peptides of sequences 1 to 49 was determined in a phage display screening assay using recombinant human MIA.

The derivatives of the peptides of sequences 1 to 49, as discussed above, can be tested for their MIA inhibitory activity using standard methods, for example, a ligand binding assay using recombinant human MIA, as discussed in Stoll *et al., op. cit* or as disclosed in Schmidt et al., Targeting melanoma metastasis and immunosuppression with a new mode of melanoma inhibitory activity (MIA) protein inhibition, 2012, PLoS ONE 7(5): e37941.

The MIA inhibitor peptide can be added as such to the composition or may be encapsulated within different polymeric materials or conjugated to different carrier substances for improving its transdermal delivery through the stratum corneum to reach the target MIA protein in the vitiliginous melanocytes, which are located in the bottom layer of the epidermis, the stratum basale, attached to the basement membrane.

Encapsulation techniques are a well-known in the art for the delivery of active ingredients.

In one embodiment, the MIA inhibitor peptide is micro-encapsulated using liposome technology.

Liposomes are commonly used in dermal formulations for improving penetration of actives through the skin layer. As is well known in the art, liposomes are spherical vesicles with sizes generally in the range between about 60 nm and 300 nm and are most often composed of phospholipids which form at least one phospholipid bilayer, but may also include other lipids. Liposomes contain hydrophilic cores in which hydrophilic actives may be encapsulated, while hydrophobic actives are incorporated in the bilayer, so liposomes are suitable carriers for both hydrophilic and lipophilic actives (Knoth et al., Nanocarrier-Based Formulations: Production and Cosmeceutic Applications, in: Cosmetic Formulation. Principles and Practice, Benson H.A.E., Roberts M.S., Rodrigues Leite-Silva V. and Walters K.A., editors, CRC Press, 2019). Liposomes may be prepared by well-known techniques; in general, the preparation methods involve mixing the membrane-forming lipids, in an organic phase, drying, subsequent hydration of the lipids and further size reduction by different mechanical treatment such as sonication, extrusion or homogenization.

In another embodiment, the MIA inhibitor is encapsulated within a microcapsule or nanocapsule made of a biodegradable polymer, as are well-known in the art, for example, as those disclosed for the delivery of the peptides of sequences 1 to 49 (WO-A-03/064457, *op.cit.*) including polyesters, polyamino acids, polyalkyl cyanoacrylates, polyphosphazenes, or polyethylene oxide. Common polymers used for encapsulation are, for example, polyvinyl alcohol (PVA), poly(lactic acid) (PLA), poly(lactic-co-glycolic acid) (PLGA) or their block co-polymers with poly(ethylene oxide) or poly(ethylene glycol). Other suitable biodegradable polymers are poly(propylene fumarate-co-ethylene glycol) [P(PF-co-EG)] block copolymer, poly-anhydride poly(fumaric-co-sebacic) anhydride, alginate, dextran, chitosan, hydroxyapatite, collagen, fibrin, hyaluronic acid, carbomers, and mixtures thereof.

In a particular embodiment, the MIA inhibitor is encapsulated within a microcapsule or nanocapsule which has attached on the outer surface a specific peptide which is a melanocortin 1 (MC1) receptor agonist and those microcapsules or nanocapsules are designed for the targeted delivery of the encapsulated MIA inhibitor to melanocytes. Said targeted capsules are disclosed in the patent application WO-A-2015/075116.

These targeted microcapsules or nanocapsules have typically a size distribution from 10 nm to 10000 nm, preferably from 50 nm to 5000 nm, more preferably from 100 nm to 1000 nm, still more preferably from 150 nm to 450 nm, and still more preferably from 180 nm to 400 nm, as disclosed in WO-A-2015/075116 *op*. *cit.* The size of the microcapsules may be determined by Scanning Electron Microscopy (SEM).

The microcapsules are preferably polymeric, generally made of one or more biodegradable polymers, for example, as those disclosed above. It is required that at least one of the polymers forming the targeted microcapsule bears carboxylic groups for binding the peptide the MC1 receptor agonist peptide to the outer surface, by coupling said carboxylic groups with the amine terminal groups in the peptide.

The microcapsules may optionally be bilayered polymeric microcapsules comprising a core polymer (or "inner layer polymer"), and an outer shell polymer (or "outer layer polymer"). For example, the core polymer may be poly (D,L-lactide-co-glycolide) (PLGA) and the outer shell polymer may be polyvinyl alcohol (PVA).

The preparation of said targeted microcapsules or nanocapsules is disclosed in WO-A-2015/075116, *op. cit.* and generally, involves the mixture of the active ingredient (MIA inhibitor peptide in this case) and the polymer(s) forming the capsule in a suitable solvent, and a further step of coupling the MC1 receptor agonist peptide, which confers affinity towards melanosomes, to the outer surface of the microcapsule or nanocapsule. This step can be performed before or after forming the capsule, preferably after forming the capsule.

The MC1 receptor agonist coupled to the outer surface of the microcapsule or nanocapsule is a peptide of the formula:

R₂-Ser-Tyr-Ser-Nle-Glu-His-DPhe-Arg-(AA)-Gly-Lys-DPro-Val-R₁

or a pharmaceutically acceptable salt or solvate thereof, wherein:
- R₁ is the radical -NH-(CH₂)₃-O-(CH₂CH₂O)ₙ-(CH₂)₃-NH₂, wherein n is an integer from 1 to 10; for example, n is 1 and R₁ derives from ethylene glycol bis (3-aminoproyl) ether (CAS No. 2997-01-5); in another example, n is 2 and R₁ derives from diethylene glycol bis (3-aminoproyl) ether, also called 4,7,10-trioxa-1,13-tridecanediamine (CAS No. 4246-51-9);
- R₂ is selected from (C₁₋₂₄ alkyl)-CO-, (C₂₋₂₄ alkenyl)-CO- and (C₆₋₁₀ aryl)-CO-; for example, R₂ is selected from acetyl, propanoyl, pentadecanoyl, hexadecanoyl and heptadecanoyl (palmitoyl); and
- AA is an amino acid containing an aromatic group; for example, AA may be selected from tryptophan, 3-(2-naphthyl)-D-alanine, 3-amino-3-(1-naphthyl)-propionic acid, 3-amino-3-(biphenyl)-propionic acid, phenylalanine, tyrosine, histidine, 5-hydroxytryptophan and L-3,4-dihydroxy-phenylalanine.

In another embodiment, the MIA inhibitor peptide is conjugated with nanoparticles, namely, with lipid nanoparticles, polymer nanoparticles, magnetic nanoparticles or metallic nanoparticles, which may act as carriers to facilitate the penetration of the MIA inhibitor through the stratum corneum.

In one particular embodiment, the MIA inhibitor peptide is conjugated with gold nanoparticles, which are described to improve penetration through skin (Gupta et al., J. Phys. Chem. B, 2016, 7133-7142). Those nanoparticles have a gold core coated with the MIA inhibitor peptide. The MIA inhibitor peptide is conjugated to the gold nanoparticles, typically by means of an electrostatic ionic bond between the negative charges of the gold surface of the nanoparticles and the positive charges of amino acids of the peptide carrying amino groups in the side chain (lysine, arginine, histidine, asparagine or glutamine). The gold nanoparticles have a size of less than 1000 nm, preferably comprised between 50 and 200 nm. The conjugates may be prepared by reducing Au (III) to Au (0) with a reducing agent and treating the gold nanoparticles obtained with the MIA inhibitor peptide, for example, as disclosed in the international patent application WO-A-2019/185696.

The amount of the MIA inhibitor peptide in the composition of the invention, either if it is in free form or encapsulated or conjugated with a carrier, as disclosed above, is typically comprised between 0.0001 % and 1%, preferably comprised between 0.0005% and 0.1%, and more preferably comprised between 0.001% and 0.05%, wherein the percentages are expressed as weight of the MIA inhibitor relative to the total weight of the composition.

### Snail secretion

Snail secretion, also known as snail slime, is a kind of mucus or bodily secretion which is produced by snails (gastropods), by glands located in the foot of the gastropod. This mucus is made of water and a complex mixture of substances, including proteoglycans, glycosaminoglycans, glycoprotein enzymes, hyaluronic acid, copper peptides, antimicrobial peptides, and metal ions; the main components are allantoin, collagen, elastin and glycolic acid (Cillia et al., Antimicrobial properties of terrestrial snail and slug mucus, J. Complement. Integr. Med., 2018, 15(3)).

This secretion may be collected and recovered from living snails, for example as disclosed in the patent US5538740 or in the US patent application US-A-2018/0064635. In general, after subjecting the gastropods to certain stress conditions, the secretion is collected, and filtered.

Snail secretion is generally in the form of a liquid, the pH is generally comprised in the range 5-8, the density is typically from about 1.0 to about 1.1 mg/ml and the dry residue is generally comprised between 0.1% and 3%, preferably comprised between 0.2% and 2%, more preferably comprised between 0.25% and 1%, and still more preferably comprised between 0.3% and 0.7%. The protein content is typically in the range 0.1-3 mg/ml.

Snail secretion is commercially available from several sources, for example, from the company Cobiosa, Spain (sold as product Poly-Hexilan PF) or from the company CENTISIA Laboratorio di Fitocosmesi di Bruno Dott.ssa Laura Francesca & C. s.a.s., Italy (sold as Prodotto 580038), among others.

Different species of gastropods may be used to obtain this secretion, including, among others, *Achatina fulica, Cornu aspersum* (or *Helix aspersa* or *Cryptomphalus aspersa or Cantareus aspersus*)*, Helix pomatia, Helix hortensis, Helix nemoralis, Helix cardidula, Helix tchthyomma, Helix fructicicola, Helix strigella, Helix fruticum, Helix bidens, Helix arbostorum, Helix rotundata, Helix aculeata, Helix pulchella, Helix personata, Helix holoserica, Helix aperta, Helix parnassia, Helix alonensis, Helix candidissima, Helix pisana,* or *Helix gualteviana.* In a particular embodiment, the snail secretion is obtained from *Cornu aspersum,* also known as *Helix aspersa, Cryptomphalus aspersa* or *Cantareus aspersus.*

The medicinal and cosmetic use of snail secretion has been disclosed in the state of the art, for example, as antimicrobial agent, for wound repair, for skin protection, or as anti-aging agent, among other uses.

The authors of the present invention have surprisingly discovered that the combination of a MIA inhibitor substance and snail secretion provides outstanding results in the treatment of vitiligo.

Indeed, as disclosed in the comparative Example 3, it was found that while a composition comprising snail secretion alone did not show any effect on vitiligo depigmented skin, the combination of this substance with MIA inhibitors provided the best anti-vitiligo effect, with notable re-pigmentation of the affected areas.

The amount of snail secretion in the composition of the invention is typically comprised between 0.5% and 15%, preferably comprised between 1% and 10%, more preferably comprised between 2% and 8% and still more preferably is about 5%, wherein the percentages are expressed as weight of the snail secretion relative to the total weight of the composition.

### Compositions of the invention

The composition of the invention comprises a MIA inhibitor peptide, snail secretion and at least one pharmaceutically acceptable excipient.

The term "excipient" or "pharmaceutically acceptable excipient" means a component of a pharmaceutical composition that is not an active ingredient, which is useful for preparing the pharmaceutical composition and is generally safe and non-toxic for human pharmaceutical use. In particular, the composition of the invention is typically intended to be used externally, topically applied over the affected skin area, so the pharmaceutically acceptable excipient is specifically "dermatologically acceptable", i.e., it is suitable and non-toxic for use in contact with human skin tissue.

In one embodiment, the composition according to present invention is for topical administration, i.e., it is to be spread over the skin in the area to be treated, namely, in the area showing vitiliginous depigmentation or prone to show vitiliginous depigmentation. Topical, dermal, or cutaneous administration are used herein interchangeably.

Any type of formulation suitable for topical administration may be used. Typically, the composition of the invention is in the form of cream, gel, cremigel, lotion, paste, foam, solution, suspension, emulsion, milk, or stick preparation, for example, which are well-known in the art. A cremigel, for example, is a type of formulation halfway between a cream and a gel: unlike a cream, a cremigel is a gelled aqueous solution or a gelled oil, but it looks opaque as a cream, usually white in colour.

Suitable carriers may be, for example, anhydrous, as mixtures of fats, waxes, animal and plant oils and solid and liquid hydrocarbons. Or the carrier may be water or an aqueous solution of hydrophilic substances. Suitably, the carrier may be in the form of an emulsion. Emulsions may be, typically, oil-in-water emulsions, water-in-oil emulsions, water-in-oil-in-water, oil-in-water-in-oil or water-in-silicone emulsions. An emulsion may generally be described as having a continuous aqueous phase (oil-in-water and water-in-oil-in-water) or a continuous oil phase (water-in-oil and oil-in-water-in-oil). The oil phase may comprise silicone oils, non-silicone oils such as paraffin hydrocarbons, fatty alcohols (e.g. stearyl alcohol, cetyl alcohol or cetostearyl alcohol), fatty acids (e.g. stearic acid, oleic acid), fatty acid esters (e.g. isopropyl myristate, isopropyl palmitate), waxes or plant oils (e.g. castor oil, canola oil, cottonseed oil, jojoba oil or arachis oil), or mixtures thereof. The aqueous phase may comprise water or a water solution of hydrophilic substances, such as alcohols (e.g. ethanol, isopropyl alcohol), polyols (e.g. glycerol, sorbitol), alpha hydroxy acids, amino acids, protein hydrolysates, simple sugars, or polysaccharides.

Emulsifiers, which are common components of emulsions, are surface-active agents (surfactants) and include non-ionic surfactants, anionic surfactants, cationic surfactants and amphoteric surfactants. Non-ionic surfactants include, among others, ethoxylated fatty alcohols, ethoxylated fatty acid esters, alkyl glucosides or alkyl oligoglucosides, ethoxylated sorbitan fatty acid esters, monoglycerol/polyglycerol fatty acid esters, ethoxylated glycerin monesters, ethoxylated polyglyceryl esters, alkyl dimethylamine oxides, or poloxamers, among others. Anionic surfactants include alkaline soaps, alkyl sulphates, alkyl ether sulphates, alkyl sulphosuccinates, alkyl phosphates, acyl sarcosinates or acyl isethionates, among others. Cationic surfactants include quaternary ammonium salts, or pyridine salts, among others. Amphoteric surfactants include imidazoline derivatives, betaines, amidobetaines and sulphobetaines.

Other common ingredients in the formulation are, for example, emollients, humectants, preservatives, viscosity controlling agents, antioxidants, pH regulators, UV filters, chelating agents, perfumes and colorants. Common emollients are, for example, paraffin hydrocarbons, silicones, fatty alcohols, fatty acids, esters of fatty acids with alcohols, triglycerides, ceramides, phospholipids and waxes. Humectants include polyhydroxy alcohols, proteins and hydroxyl acids. Common preservatives include sorbic acid and its salts, benzoic acid and its salts, parabens, imidazolidinyl urea, diazolidinyl urea, DMDM hydantoin, sodium hydroxymethylglycinate, methylchloroisothiazolinone/methylisothiazolinone, benzyl alcohol and 2-phenoxyethanol, among others. Other additives may also be added for controlling the viscosity of the formulation, for example, xanthan gum, gellan gum, acacia, carrageenans, chitosan, collagen, tragacanth, pectin, starch derivatives, carbomers, cellulose derivatives (hydroxyethylcellulose, hydroxypropylcellulose, hydroxypropyl methylcellulose, methylcellulose, ethylcellulose, or carboxymethylcellulose, for example), polyamides, glutamides, colloidal silica or waxes (e.g. beeswax or vegetable waxes), among others.

Some ingredients of the composition may act as penetration enhancers, to facilitate the penetration of the active ingredients throughout the stratum corneum. Common penetration enhancers are, for example, short chain alcohols such as ethanol or isopropyl alcohol; long chain alcohols such as decanol, hexanol, lauryl alcohol, myristyl alcohol, octanol, octyldodecanol or oleyl alcohol; cyclic amides such as azone; esters such as ethyl acetate octyl salicylate, padimate O, ethyl oleate, glyceryl monoleate, glyceryl monocaprate, glyceryl tricaprylate, isopropyl myristate, isopropyl palmitate, propylene glycol monolaurate, or propylene glycol monocaprylate; ether alcohols such as Transcutol^{®}; fatty acids such as lauric acid, linoleic acid, linolenic acid, myristic acid, oleic acid, palmitic acid, stearic acid or isostearic acid; glycols such as dipropylene glycol, propylene glycol, 1,2-butylene glycol or 1,3- butylene glycol; pyrrolidones such as *N*-methyl-2-pyrrolidone or 2-pyrrolidone; sulphoxides such as decylmethyl sulphoxide or dimethyl sulphoxide; anionic surfactants; cationic surfactants; non-ionic surfactants; terpenes such as eugenol D-limonene, menthol, menthone, farnesol or neridol (as disclosed, for example, in Lane M.E., Skin penetration enhancers, Int. J. Pharm., 2013, 447, 12-21; or in Haque et al., Chemical enhancer: a simplistic way to modulate barrier function of the stratum corneum, Adv. Pharm. Bull., 2018, 8(2), 169-179).

The composition of the invention has typically a slightly acidic pH, close to the physiological pH of the skin. Common acidity regulators are organic acids, including hydroxy acids and fatty acids. Some of the most common pH regulators in cosmetic emulsions are hydroxy acids such as lactic or citric acid.

One excipient can perform more than one function. The above cited ingredients, as well as many others suitable dermatological formulation excipients, are well-known to the skilled formulator of topical compositions. Such ingredients are commercially available from several companies, such as Comercial Química Massó, SA, Evonik, DuPont or Dow Corning, among others.

The preparation of the topical composition is made according to procedures well-known to the skilled in the formulation of dermatological compositions, generally involving simple steps of mixing, and optionally heating the component ingredients.

A cream, for example, can be typically prepared by warming the oily phase and the aqueous phase separately to a temperature of about 60 to 80° C, and then mixing under stirring to prepare the emulsion.

The main ingredients of the compositions and the procedures for preparing them are entirely analogous to those employed for the preparation of topical cosmetic formulations, and are described, for example, in the book: Cosmetic Formulation. Principles and Practice, Benson H.A.E., Roberts M.S., Rodrigues Leite-Silva V. and Walters K.A., editors, CRC Press, 2019, or in other similar reference books. Also, regulated cosmetic substances and ingredients are disclosed in the European Commission database "Coslng" (https://ec.europa.eu/arowth/tools-databases/cosing)

Typically, for example, a cream, gel, or lotion base formulation is first prepared, using standard ingredients and procedures well-known in the art, and then snail secretion and the MIA inhibitor peptide (either in free form or encapsulated) are added and thoroughly mixed, and the pH of the composition is finally adjusted.

### Use of the composition

As shown in the prospective clinical trials described in Examples 3 and 4, the composition of the invention is outstandingly effective for treating vitiligo, with apparent re-pigmentation of the treated depigmented areas.

Another aspect of the invention is, therefore, the composition of the invention for use in the prevention and/or treatment of vitiligo.

Another aspect of the invention is a method for the prevention and/or treatment of vitiligo in a patient in need thereof comprising the steps of applying a therapeutically effective amount of the composition of the invention.

The treatment of vitiligo is understood as the procedure aimed to improve vitiligo symptoms, namely, to partially or totally restore the normal pigmentation of the depigmented or hypopigmented skin patches.

In one embodiment, the prevention and/or treatment of vitiligo relates to the prevention and/or treatment of non-segmental vitiligo.

The prevention of vitiligo is aimed to prevent the onset of vitiligo symptoms and is addressed to subjects which are known to be prone to suffer from vitiligo, either for having been early diagnosed, before the appearance of symptoms, for example, by detecting the presence of MIA protein in the melanocytes, or for having some known genetic predisposition to suffer from this disease. The prevention of vitiligo also includes the treatment of patients who had previously suffered from vitiligo and have recovered from the disease, i.e., who have achieved re-pigmentation of the vitiliginous depigmented skin, to prevent them from suffering again from vitiligo symptoms.

Typically, the composition of the invention is administered topically, i.e., it is spread over the vitiliginous skin patches.

In one embodiment, the composition of the invention may be delivered transdermally, for example, via iontophoresis, ultrasound or using microneedles (Escobar-Chávez et al. Microneedles: a valuable physical enhancer to increase transdermal drug delivery, J. Clin. Pharmacol., 2011, 51(7), 964-977).

The amount of the composition to be used, considered to be "therapeutically effective" can widely vary depending on many factors, for example, the severity and progression stage of the disease, or the specific formulation used. The amount can be easily adjusted in each case by the skilled practitioner. In general, the amount of the topical composition applied to the affected skin may range from about 0.1 mg of composition for each cm² of skin surface area (mg/cm²) to about 100 mg/cm², preferably from about 5 mg/cm² to about 10 mg/cm².

The compositions of the present invention may be applied topically once or more times daily, for example twice, three times or four times daily. The composition is typically applied by spreading it over the skin, generally, only over the affected skin area. The duration of the treatment may be adjusted according to the patient's evolution. Typically, the treatment may be maintained for several weeks, for example 1, 2, 3 or 4 weeks, or several months, for example, 1 to 12 months, or even more, depending on the severity of vitiligo symptoms and the evolution of the disease. The duration of the treatment can be easily adjusted by the skilled practitioner.

Advantageously, the treatment with the composition of the invention may be combined with phototherapy, for example, by simple sunlight exposure or by the use of sunbeds, to stimulate melanocyte formation and migration and thus boosting the recovery. Indeed, as shown in Example 4, the composition of the invention, combined with sun exposure, lead to almost complete recovery, i.e., complete re-pigmentation of the skin.

Another aspect of the invention is, therefore, a method for the prevention and/or treatment of vitiligo in a patient in need thereof comprising the steps of applying a therapeutically effective amount of the composition of the invention and exposing the treated skin area to phototherapy.

The term "phototherapy" or "light therapy" as used herein includes exposure to sunlight or to radiation of specific wavelengths, for example, UV radiation, including UVA and UVB radiation. Therefore, "phototherapy" can be performed by simple exposure to sunlight, or using sunbeds, or using a radiation emitting device, in particular, a UV emitting device.

The duration of the phototherapy is not decisive, and may range, for example, from about 10 minutes to about 2 hours daily, or on alternate days, or twice or three times weekly, among other suitable options.

Vitiligo can affect any area of skin, so the composition may be applied to any area of affected (depigmented) skin. The areas of skin most commonly affected by vitiligo and, therefore, more in need to be treated, are hands and face, around body openings (the eyes, nostrils, mouth, umbilicus and genital regions), and within body folds such as the underarms and groin.

In one embodiment, the use of the composition of the invention for treating vitiligo may be combined with the use of antioxidants, in particular, when the therapy is combined with phototherapy, in order to reduce the possible negative effects of the radiation exposure.

The antioxidants are typically for oral use. Suitable antioxidants are, among others, vitamin E, vitamin C, carotenoids (such as lycopene), beta-carotene, alpha-carotene, green tea extract, zinc, selenium, *Polypodium leucotomos* extract, among others, or combinations thereof.

They can be formulated as a dietary oral supplement, typically as tablets, powders or capsules, for example, using standard excipients and preparation process, known in the art.

In one embodiment, the invention relates to a kit comprising the composition of the invention and a dietary supplement comprising an antioxidant.

In another embodiment, the invention relates to a kit comprising the composition of the invention and a dietary supplement comprising an antioxidant for use in the prevention and/or treatment of vitiligo.

Another aspect of the invention is also a method for the prevention and/or treatment of vitiligo in a patient in need thereof comprising the steps of applying a therapeutically effective amount of the composition of the invention, exposing the treated skin area to phototherapy and administering to the patient an antioxidant.

The present invention may be defined according to the following embodiments:
1. A pharmaceutical composition comprising:
   (a) a MIA inhibitor peptide which has a sequence selected from SEQ ID NO: 1 to SEQ ID NO: 49, or a derivative thereof;
   (b) snail secretion; and
   (c) at least one pharmaceutically acceptable excipient.
2.- The pharmaceutical composition according to embodiment 1, characterized in that the sequence is selected from SEQ ID NOs 12-20 and 27-49, and preferably is selected from SEQ ID NOs 34, 37, 39-43 and 45-47.
3.- The pharmaceutical composition according to embodiment 2, characterized in that the sequence is SEQ ID NO: 40.
4.- The pharmaceutical composition according to any one of embodiments 1 to 3, characterized in that the derivative is selected from:
   i) sequences wherein one additional amino acid is added or wherein one amino acid is deleted;
   ii) sequences wherein one or more amino acids, preferably no more than 3 amino acids, more preferably no more than 2 amino acids, and more preferably only one amino acid is/are substituted by another natural or non-natural amino acid; and
   iii) a sequence as defined in SEQ ID NOs 1-49 or a derivative thereof according to i) or ii), wherein at least one amino acid is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation; preferably, the sequence is acetylated at the N-terminus and/or amidated at the C-terminus; and more preferably the sequence is acetylated at the N-terminus and amidated at the N-terminus.
5.- The pharmaceutical composition according to any one of embodiments 1 to 3, characterized in that the derivative consists in that at least one amino acid of the sequence is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation; preferably, the sequence is acetylated at the N-terminus and/or amidated at the C-terminus; and more preferably the sequence is acetylated at the N-terminus and amidated at the N-terminus.
6.- The pharmaceutical composition according to any one of embodiments 1 to 5, characterized in that the MIA inhibitor peptide is encapsulated or is conjugated with a carrier nanoparticle.
7.- The pharmaceutical composition according to embodiment 6, characterized in that the MIA inhibitor peptide is encapsulated within a microcapsule or nanocapsule which has attached on the outer surface a specific peptide which is a melanocortin 1 (MC1) receptor agonist.
8.- The pharmaceutical composition according to embodiment 7, characterized in that the MC1 receptor agonist is a peptide of the formula:

   R₂-Ser-Tyr-Ser-Nle-Glu-His-DPhe-Arg-(AA)-Gly-Lys-DPro-Val-R₁

   or a pharmaceutically acceptable salt or solvate thereof, wherein:
   - R₁ is the radical -NH-(CH₂)₃-O-(CH₂CH₂O)ₙ-(CH₂)₃-NH₂, wherein n is an integer from 1 to 10; for example, n is 1 and R₁ derives from ethylene glycol bis (3-aminoproyl) ether (CAS No. 2997-01-5); in another example, n is 2 and R₁ derives from diethylene glycol bis (3-aminoproyl) ether, also called 4,7,10-trioxa-1,13-tridecanediamine (CAS No. 4246-51-9);
   - R₂ is selected from (C₁₋₂₄ alkyl)-CO-, (C₂₋₂₄ alkenyl)-CO- and (C₆₋₁₀ aryl)-CO-; for example, R₂ is selected from acetyl, propanoyl, pentadecanoyl, hexadecanoyl and heptadecanoyl (palmitoyl); and
   - AA is an amino acid containing an aromatic group; for example, AA may be selected from tryptophan, 3-(2-naphthyl)-D-alanine, 3-amino-3-(1-naphthyl)-propionic acid, 3-amino-3-(biphenyl)-propionic acid, phenylalanine, tyrosine, histidine, 5-hydroxytryptophan and L-3,4-dihydroxy-phenylalanine.
9.- The pharmaceutical composition according to embodiment 6, characterized in that the MIA inhibitor peptide is conjugated with gold nanoparticles.
10.- The pharmaceutical composition according to any one of embodiments 1 to 9, characterized in that the amount of the MIA inhibitor peptide in the composition is comprised between 0.0001% and 1%, preferably comprised between 0.0005% and 0.1%, and more preferably comprised between 0.001% and 0.05%, wherein the percentages are expressed as weight of the MIA inhibitor relative to the total weight of the composition.
11.- The pharmaceutical composition according to any one of embodiments 1 to 10, characterized in that the amount of snail secretion in the composition is comprised between 0.5% and 15%, preferably comprised between 1% and 10%, more preferably comprised between 2% and 8% and still more preferably is about 5%, wherein the percentages are expressed as weight of the snail secretion relative to the total weight of the composition.
12.- The pharmaceutical composition according to any one of embodiments 1 to 11, characterized in that it is for topical use.
13.- The pharmaceutical composition according to embodiment 12, characterized in that it is selected from cream, gel, cremigel, lotion, paste, foam, solution, suspension, emulsion, milk, and stick, preferably is selected from cream, gel and cremigel, more preferably is selected from cream and gel.
14.- A kit comprising the pharmaceutical composition according to any one of embodiments 1 to 13 and a dietary supplement comprising an antioxidant.
15.- The pharmaceutical composition according to any one of embodiments 1 to 13 or the kit according to embodiment 14 for use in the prevention and/or treatment of vitiligo.
16.- The pharmaceutical composition or the kit for use according to embodiment 15, characterized in that vitiligo is non-segmental vitiligo.
17.- A method for the prevention and/or treatment of vitiligo in a patient in need thereof comprising the steps of applying a therapeutically effective amount of the composition of embodiments 1 to 13.
18.- A method for the prevention and/or treatment of vitiligo in a patient in need thereof comprising the steps of applying a therapeutically effective amount of the composition of embodiments 1 to 13 and exposing the treated skin area to phototherapy.
19.- The method according to embodiment 18, wherein phototherapy includes exposure to sunlight or to radiation of specific wavelengths, for example, UV radiation, including UVA and UVB radiation.
20.- The method according to embodiments 18 or 19, wherein the method includes an additional step of administering to the patient an antioxidant.
21.- The method according to any one of embodiments 17 to 20, wherein vitiligo is non-segmental vitiligo.

### Examples

### Example 1 MIA inhibitor peptide

The MIA inhibitor of SEQ ID NO: 40 was used for preparing the compositions of Example 2.

### Preparative example 1: encapsulated MIA inhibitor peptide

Targeted microcapsules of the MIA inhibitor peptide were prepared as bilayered microcapsules wherein the inner polymer was (D,L-lactide-co-glycolide) (PLGA) and the outer polymer was polyvinyl alcohol (PVA), said capsules had attached to the surface the melanocortin 1 (MC1) receptor agonist peptide Palmitoyl-Ser-Tyr-Ser-Nle-Glu-His-DPhe-Arg-Trp-Gly-Lys-DPro-Val-NH-(CH₂)₃-(OCH₂CH₂)₂-CH₂-NH₂ (SEQ ID NO: 50) which was coupled to the capsule by means of an amide bond between the amino terminal group of the peptide and the carboxylic groups available on the surface of the capsule from PLGA. Said microcapsules were prepared using a method analogous to that disclosed in Example 1 of the international patent application WO-A-2015/075116.

The proportion of the MIA inhibitor peptide in the obtained capsules was about 35 wt%, referred to the total weight of the capsules.

Those targeted capsules were incorporated into the composition of the invention (see Example 2) in the form of an aqueous solution comprising about 0.1 wt% of the capsules (the solution also comprised xanthan gum, phenoxyethanol, caprylyl glycol, glycerin, glyceryl caprylate and phenylpropanol).

### Preparative example 2: gold-anti-MIA peptide conjugate

The MIA inhibitor peptide (SEQ ID NO: 40) was conjugated with gold nanoparticles following a procedure analogous to that disclosed in Examples 1 and 2 of the international patent application WO-A-2019/185696, by first preparing the gold nanoparticles by reducing a gold salt (HAuCl₄) with sodium citrate, and then adding the MIA inhibitor peptide.

The proportion of the MIA inhibitor peptide in the gold conjugate was about 25 wt%, referred to the total weight of the gold-MIA inhibitor conjugate.

This conjugate was incorporated into the composition of the invention (see Example 2) in the form of an aqueous dispersion comprising about 0.5 wt% of the conjugate (the dispersion also comprised xanthan gum and phenoxyethanol).

### Example 2 Preparation of a composition according to the invention

A composition according to the present invention was prepared using the components listed in the following table:

| **Ingredients** | | **Weight%** | | | |
|---|---|---|---|---|---|
| | | **A** | **B** | **C** | **D** |
| A1 | Emulium^{®} Mellifera MB (polyglyceryl-6 distearate, jojoba esters, polyglyceryl-3 beeswax and cetyl alcohol) | 5.0 | 5.0 | 5.0 | 5.0 |
| A2 | Isohexadecane | 2.0 | 2.0 | 2.0 | 2.0 |
| A3 | Isopropyl isostearate | 1.0 | 1.0 | 1.0 | 1.0 |
| A4 | Isononyl Isononanoate | 2.0 | 2.0 | 2.0 | 2.0 |
| A5 | Tocopheryl acetate | 0.2 | 0.2 | 0.2 | 0.2 |
| B1 | Deionized water | 63.9 | 71.9 | 68.9 | 63.9 |
| B2 | Potassium cetyl phosphate | 0.3 | 0.3 | 0.3 | 0.3 |
| B3 | Anhydrous betaine extracted from sugar beet | 2.0 | 2.0 | 2.0 | 2.0 |
| B4 | Preservatives (methylpropanediol, caprylyl glycol and phenylpropanol) | 2.0 | 2.0 | 2.0 | 2.0 |
| B5 | Phenoxyethanol | 0.5 | 0.5 | 0.5 | 0.5 |
| B6 | Disodium EDTA | 0.1 | 0.1 | 0.1 | 0.1 |
| B7 | Glycerin | 2.0 | 2.0 | 2.0 | 2.0 |
| B8 | Xanthan gum | 0.2 | 0.2 | 0.2 | 0.2 |
| C1 | SEPIPLUS^{™} 400 (Polyacrylate-13, Polyisobutene and Polysorbate 20) | 0.5 | 0.5 | 0.5 | 0.5 |
| C2 | Cyclopentasiloxane | 2.0 | 2.0 | 2.0 | 2.0 |
| C3 | Dimethicone | 1.0 | 1.0 | 1.0 | 1.0 |
| D1 | Snail secretion | 5.0 | 5.0 | 5.0 | 5.0 |
| D2a | Anti-MIA peptide encapsulated 0.1 wt% | 10.0 | - | - | - |
| D2b | Anti-MIA peptide-gold conjugate 1 wt% | - | 2.0 | - | - |
| D2c | Anti-MIA peptide 0.1 wt% | - | - | 5.0 | 10.0 |
| D3 | Perfume | 0.3 | 0.3 | 0.3 | 0.3 |
| | Total | 100.0 | 100.0 | 100.0 | 100.0 |

The MIA inhibitor ("anti-MIA") peptide of SEQ ID NO: 40 was used for preparing the compositions. As disclosed in Example 1, it was added either:
- enclosed within melanocyte-targeted capsules (component D2a)
- as a conjugate with gold nanoparticles (component D2b)
- as free peptide (component D2c)

Components D2a and D2b were added as a solution/dispersion, as disclosed in Example 1. Component D2c (free MIA-inhibitor peptide) was added as an aqueous solution (0.1 wt%).

Therefore, the proportion of the MIA inhibitor peptide in the compositions A, B, C and D was about 0.0035% (35 ppm), 0.0025% (25 ppm), 0.005% (50 ppm) and 0.01% (100 ppm), respectively.

For preparing the composition, first components A1-A6 were heated to about 70° C to 75 °C and thoroughly mixed to obtain a first liquid phase ("phase A"). Components B1-B8 were separately mixed to form a solution, which was heated to about 70° C to 75° C. Phase A was added to this solution and was emulsified in a high-shear Ultra-Turrax mixer at 8000 rpm for about 3 minutes and the emulsion was then stirred in a blade mixer at 300 rpm for about 15 minutes. The components C1-C3 were added at 60 °C, the mixture was allowed to cool down to room temperature and components D1, one of D2a/D2b/D2c and D3 were finally added. The final pH of the composition was checked to be in the range 5.5-6.5 (or otherwise adjusted with 10% citric acid or 10% NaOH).

### Example 3 Comparative study to assess the efficacy of the combination MIA inhibitor peptide - snail secretion

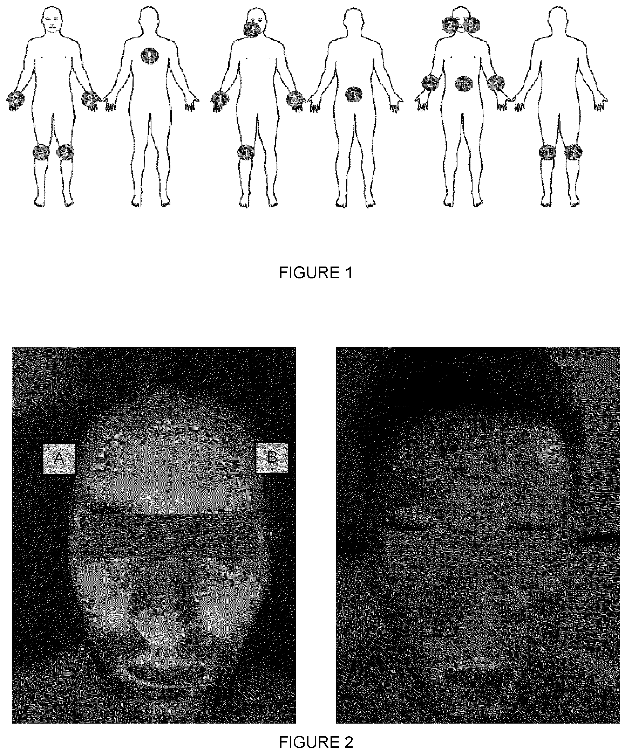

In the assays performed, up to three compositions were comparatively tested in patients suffering from vitiligo. For obtaining reliable comparative data, all three compositions were tested in each patient, by using them at different depigmented areas. Figure 1 shows some examples of randomized treated sites, for example for three hypothetical tested compositions (1, 2 and 3).

For assessing the effect of combining the MIA inhibitor peptide and the snail secretion, the following compositions were compared:

| **Active ingredients** | **Weight%** | | |
|---|---|---|---|
| | **Comparative-I** | **Comparative-II** | **C** |
| Snail secretion | 5 | - | 5 |
| Anti-MIA peptide 0.1 wt% | - | 5 | 5 |

Comparative-I and Comparative-II compositions were prepared using the same excipients A1-A5, B1-B8, C1-C3 and D3 as disclosed in Example 1, in the same amounts, and adjusting the amount of water. Comparative-I formulation comprised 5% of snail secretion (D1) but no MIA inhibitor, and Comparative-II formulation comprised 5% of MIA-inhibitor peptide 0.1% solution (i.e. 50 ppm) but no snail secretion. Formula C comprised both 5% of snail secretion and 50 ppm of the MIA inhibitor peptide. The preparation of the comparative formulas was analogous as disclosed in Example 2.

In the trial, 3 subjects suffering from vitiligo (2 females and 1 male) were treated with the three formulations. Different anatomic sites were treated, including face, chest, armpit, arm, elbow, forearm, wrist, hand, belly, back, buttock, thighs, knee and leg.

The treatment consisted in applying the cream over the treated area twice a day for a period of 3 months.

After the treatment was completed, the efficacy was evaluated by assessing the percentage of re-pigmentation of the treated areas, using a 0-10 scale, wherein 0 means no effect and 10 means 100% of re-pigmentation of the affected area.

The results are shown in the following table:

| **Composition** | **Efficacy** |
|---|---|
| Comparative-1 | 0 |
| Comparative-2 | 2 |
| C | 6 |

Thus, the composition comprising snail secretion alone did not provide any improvement. The composition comprising the MIA inhibitor alone provided only moderate re-pigmentation (of about 20%) of the vitiliginous patches. The composition of the invention (composition C) comprising both snail secretion and MIA inhibitor provided the best results with almost 60% re-pigmentation of the affected skin areas.

### Example 4 Clinical study to assess the efficacy of the composition of the invention

A prospective clinical study was performed to assess the effectiveness of Formulations A, B and C (Example2).

11 patients (7 females and 4 males, aged 18 to 60) suffering from non-segmental vitiligo were enrolled. 16 different anatomic sites were treated, namely, around the eyes, around the mouth, neck, chest, armpit, arm, elbow, forearm, wrist, hand, belly, back, buttock, thighs, knee and leg.

The three formulations were tested in all the patients, by using them at different depigmented areas. The treated sites were randomized, for example, as shown in Figure 1.

The treatment consisted in applying the cream over the treated area twice a day for a period of 5 months. During the treatment, the patients were requested to get some sun exposure or to use sunbeds. The patients were also requested to take an oral anti-oxidant composition (comprising *Polypodium leucotomos* extract, green tea extract and vitamin E) in order to neutralize any possible adverse effect derived from sun exposure.

9 patients (i.e 82% of the patients) showed at least partial re-pigmentation with all the three treatments assayed, while 2 of the patients were non-respondent. Most of respondents showed outstanding re-pigmentation of the vitiliginous depigmented areas, with a degree of re-pigmentation of 90% or higher.

No significant differences were found between the three tested compositions (A, B and C).

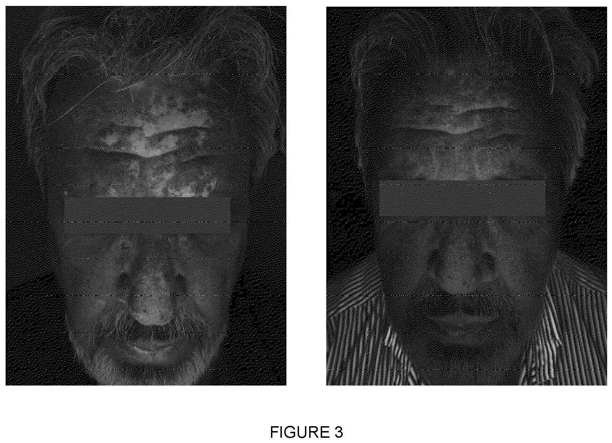

For Example, Figure 2 shows the face of one patient, before and after the treatment, who was treated with composition A on the left side and composition B on the right side. Figure 3 shows the face of another patient, before and after the treatment, who was treated with composition C.

## Claims

1. A pharmaceutical composition comprising:
(a) a MIA inhibitor peptide which has a sequence selected from SEQ ID NO: 1 to SEQ ID NO: 49, or a derivative thereof;
(b) snail secretion; and
(c) at least one pharmaceutically acceptable excipient.

2. The pharmaceutical composition according to claim 1, **characterized in that** the sequence is selected from SEQ ID NOs 12-20 and 27-49.

3. The pharmaceutical composition according to claim 2, **characterized in that** the sequence is SEQ ID NO: 40.

4. The pharmaceutical composition according to any one of claims 1 to 3, **characterized in that** the derivative is selected from:
i) sequences wherein one additional amino acid is added or wherein one amino acid is deleted;
ii) sequences wherein one or more amino acids, preferably no more than 3 amino acids, are substituted by another natural or non-natural amino acid; and
iii) a sequence as defined in SEQ ID NOs 1-49 or a derivative thereof according to i) or ii), wherein at least one amino acid is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation.

5. The pharmaceutical composition according to any one of claims 1 to 3, **characterized in that** the derivative consists **in that** at least one amino acid of the sequence is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation; preferably, the sequence is acetylated at the N-terminus and/or amidated at the C-terminus; and more preferably the sequence is acetylated at the N-terminus and amidated at the N-terminus.

6. The pharmaceutical composition according to any one of claims 1 to 5, **characterized in that** the MIA inhibitor peptide is encapsulated or is conjugated with a carrier nanoparticle.

7. The pharmaceutical composition according to claim 6, **characterized in that** the MIA inhibitor peptide is encapsulated within a microcapsule or nanocapsule which has attached on the outer surface a specific peptide which is a melanocortin 1 (MC1) receptor agonist.

8. The pharmaceutical composition according to claim 6, **characterized in that** the MIA inhibitor peptide is conjugated with gold nanoparticles.

9. The pharmaceutical composition according to any one of claims 1 to 8, **characterized in that** the amount of the MIA inhibitor peptide in the composition is comprised between 0.0001 % and 1%, expressed as weight of the MIA inhibitor relative to the total weight of the composition.

10. The pharmaceutical composition according to claim 9, **characterized in that** the amount of the MIA inhibitor peptide in the composition is comprised between 0.0005% and 0.1%, expressed as weight of the MIA inhibitor relative to the total weight of the composition.

11. The pharmaceutical composition according to any one of claims 1 to 10, **characterized in that** the amount of snail secretion in the composition is comprised between 0.5% and 15%, expressed as weight of the snail secretion relative to the total weight of the composition.

12. The pharmaceutical composition according to any one of claims 1 to 11, **characterized in that** it is for topical use.

13. The pharmaceutical composition according to claim 12, **characterized in that** it is selected from a cream and a gel.

14. A kit comprising the pharmaceutical composition according to any one of claims 1 to 13 and a dietary supplement comprising an antioxidant.

15. The pharmaceutical composition according to any one of claims 1 to 13 or the kit according to claim 14 for use in the prevention and/or treatment of vitiligo.
